# EUROPÄISCHE PATENTSCHRIFT

(11) **EP 3 209 386 B1**
(45) Veröffentlichungstag und Bekanntmachung des Hinweises auf die Patenterteilung: **12.12.2018**
(21) Anmeldenummer: 15784024.0
(22) Anmeldetag: 21.10.2015
(51) Int. Cl.: A61Q 11/00, A61K 8/25

(54) **DENTALE POLIER- UND REINIGUNGSPASTE**
DENTAL PASTE FOR CLEANING AND POLISHING
DENTIFRICE POUR NETTOYER ET POLIR LES DENTS

(30) Priorität: 24.10.2014 DE 102014115518
(43) Veröffentlichungstag der Anmeldung: 30.08.2017
(73) Patentinhaber: Orochemie GmbH & Co. KG, 70806 Kornwestheim (DE)
(72) Erfinder: SCHELLHAMMER, Uwe, 71634 Ludwigsburg (DE); HARTL, Jennifer, 71332 Waiblingen (DE)
(74) Vertreter: Witte, Weller & Partner Patentanwälte mbB
(86) Internationale Anmeldenummer: PCT/EP2015/074303
(87) Internationale Veröffentlichungsnummer: WO 2016/062741

(56) Entgegenhaltungen:
- EP-A2- 0 012 008
- US-A- 4 038 380
- US-A- 4 418 053

## Beschreibung

Die vorliegende Erfindung betrifft eine Zusammensetzung zur Dentalpolitur und/oder -reinigung, welche ein Calciumsilikat als Abrasivkörper enthält.

Die Politur von Zahnoberflächen und Implantaten gehört bereits seit längerem zu den üblicherweise durchgeführten Zahnpflegebehandlungen, um glatte und saubere Zähne zu erhalten, wobei hier nicht nur ästhetische Gesichtspunkte im Vordergrund stehen, sondern auch hygienische Motive: Mit der Politur werden nicht nur exogene Verfärbungen entfernt, die durch Nikotin-, Tee-, und Rotweinkonsum, etc. hervorgerufen werden, und die *per se* keine Zahnerkrankungen auslösen; auch supragingivale - wie auch subgingivale - Zahnbeläge lassen sich mit einer geeigneten Politur entfernen, weshalb diese auch zum therapeutischen Polieren eingesetzt werden.

Die üblicherweise eingesetzten Polierpasten bzw. -zusammensetzungen enthalten Abrasivkörper unterschiedlicher Partikelgröße, wobei hierbei zu beachten ist, dass mit gröberen Abrasivkörper zwar eine größere Reinigungswirkung erzielt werden kann, dass aber andererseits gleichzeitig auch deren Abrasivität erhöht wird. Im schlimmsten Fall können gröbere Putzkörper sogar Kratzer in die Zahnoberfläche schneiden. Umgekehrt wird mit feineren Putzkörpern eine verminderte Reinigungswirkung erreicht, die Zahnoberfläche aber geschont.

Die gegenwärtig in der Zahnreinigung bzw. -politur hierfür eingesetzten Polierpasten sollen dabei insbesondere die folgenden idealen, klinischen Wirkungen erfüllen, nämlich eine hohe Reinigungseffizienz, eine geringe bzw. zahnschonende Abrasivität, sowie eine hohe Polierwirkung, wobei mit letzterer Zahnoberflächen "glänzend" veredelt werden sollen.

Es hat sich allerdings in der Praxis gezeigt, dass sich diese Anforderungen nur schwer erfüllen lassen, da sich regelmäßig die Bestandteile der Zusammensetzungen beeinflussen und sich teilweise gegenseitig aufheben. Auch werden mit Polierpasten mit hoher Reinigungseffizienz aufgrund deren hoher Abrasivität die damit behandelten Zähne sensibilisiert, was wiederum zu einer hohen Schmerzempfindlichkeit führt. Trotz des Bemühens, die therapeutische Wirkung der Polierpasten zu optimieren, besteht nach wie vor das Problem, dass mit der Politur zwangsläufig Teile der Zahnoberfläche abgetragen werden.

Verschiedene Substanzen werden gegenwärtig als Putz- bzw. Abrasivkörper in dentalen Reinigungs- und dentalen Polierpasten verwendet, um Verfärbungen, Plaque und sonstige weiche oder harte Ablagerungen auf bzw. von Zahnoberflächen zu entfernen.

So wurden und werden in Polier- und Reinigungspasten als Abrasivkörper u.a. (Di-)Calciumphosphat, Calciumpyrophosphat, Calciumcarbonate und Calciumsilicate eingesetzt, wobei bei dem Einsatz dieser Substanzen insbesondere im Hinblick auf die mittlere Partikelgröße und deren mengenmäßigen Anteil in der Zusammensetzung unterschiedliche Ansätze getestet wurden. Dabei haben sich insbesondere (Di)-Calciumphosphat und Calciumpyrophosphat als zu weich erwiesen.

So ist im Stand der Technik darüber hinaus bspw. die US 4,038,380 bekannt, gemäß welcher Calciumsilicat als Reinigungs- und Poliersubstanz in einer Zahnreinigungszusammensetzung eingesetzt wird, wobei die Calciumsilicat-Partikel vorzugsweise eine Größe von 2 bis 15 µm und eine Mohs'sche Härte von 4,5 aufweisen. Die EP 0 012 008 A2 offenbart eine Zahnpasta-Zusammensetzung, die Wollastonit in einer Menge von 3.16 Gew.-% enthält, sowie als Hauptbestandteil Sorbitol.

Ferner sind aus der WO 2013/041419 orale Reinigungszusammensetzungen bekannt, bei welchen Calciumcarbonat-Abrasivkörper eingesetzt werden.

Die im Stand der Technik bekannten Polierpasten erfüllen die o.g. klinischen Anforderungen jedoch nach wie vor nicht in einer befriedigenden Weise, da sie sich entweder durch eine hohe Abrasivität - bei gleichzeitiger guter Reinigungswirkung - auszeichnen, oder aber durch eine geringe Abrasivität und wiederum schlechterer Reinigungswirkung, die zur mangelnden Akzeptanz bei entsprechend behandelten Patienten führt.

Nach wie vor besteht also das Bedürfnis, eine optimale Rezeptur für eine Zusammensetzung zu finden, die den klinischen Anforderungen genügt, ohne den Zähnen zu schaden. Aufgabe der vorliegenden Erfindung ist es daher, eine solche Zusammensetzung bereitzustellen, um die Nachteile des Standes der Technik zu überwinden.

Die der Erfindung zugrunde liegende Aufgabe wird erfindungsgemäß gelöst durch eine Zusammensetzung, die 5 bis 70 Gew.-% eines nadelförmigen kristallinen Calciumsilikats als ein erster Abrasivkörper aufweist, worin das nadelförmige kristalline Calciumsilikat ein Längen-Breiten-Verhältnis von 3 : 1 bis 20 : 1, wobei das nadelförmige kristalline Calciumsilikat Wollastonit ist, und wobei ein Polierkörper vorliegt, der ausgewählt ist aus Hydroxylapatit, Feldspat, Bimsstein, Eisenglimmer.

Die Aufgabe wird ferner durch die Verwendung der erfindungsgemäßen Zusammensetzung zur Dentalpolitur und/oder Dentalreinigung gelöst, sowie durch ein entsprechendes Politur-/Reinigungsverfahren, bei welchem die erfindungsgemäße Zusammensetzung eingesetzt wird.

Mit der erfindungsgemäßen Zusammensetzung bzw. deren Verwendung zur Politur und/oder Reinigung wird eine schonende Behandlung mit gleichzeitig gutem Reinigungseffekt erreicht. Vorteilhaft sind die Abrasivkörper dabei zu Beginn der ZahnReinigung/Politur nadelförmig, und besitzen daher eine gute Abtragsleistung, wobei im weiteren Verlauf der Reinigung/Politur die partikelförmigen zuvor nadelförmigen Abrasivkörper stark verrunden, also ihre Partikelform insbesondere in Bezug auf ihre Länge verändern; dadurch wird vom Reinigungsvorgang direkt in den Poliervorgang übergegangen, da die verkleinerten bzw. abgerundeten Calciumsilikat-Partikel keinen bzw. im Wesentlichen keinen oder nur einen geringen Abrasiveffekt zeigen, aber eine gute Poliereigenschaft besitzen, und damit einen guten Glanz erzielen.

Vorliegend, wie auch allgemein im betreffenden Gebiet, wird unter einem "Abrasivkörper" jede partikelförmige Substanz verstanden, die aufgrund ihrer Eigenschaften Partikelförmigkeit, Volumen und Härte dazu in der Lage ist, weiche und/oder harte Ablagerungen von Zahnoberflächen abzutragen.

Mit der o.g. Definition der Nadelförmigkeit, d.h. mit der Angabe des Verhältnisses der Länge zur Breite der Partikel des Abrasivkörpers, wird eine Definition für die erfindungsgemäß einzusetzenden Abrasivkörper gegeben. Dementsprechend bedeutet "nadelförmig", dass die Länge des Abrasivkörpers immer größer als seine Breite ist, und er mindestens dreimal so lang wie breit ist, und dadurch die Form eines mehr oder weniger feinen und spitzen Partikels erhält, wobei als "Spitze" lediglich die beiden Enden der Längsseite des Partikels zu verstehen sind, deren Durchmesser kleiner sein kann als ein mittiger Bereich der Nadel.

Die nadelförmigen Calciumsilikate, die als Abrasivkörper eingesetzt werden, können dabei eine beispielhafte Partikelgröße bzw. Partikellänge (D50) von ca. 5 µm bis 40 µm, insbesondere 20 - 30 µm, besitzen.

Erfindungsgemäß weist dabei die Zusammensetzung zur Dentalpolitur und/oder -reinigung 5 bis 70 Gew.-% des ersten Abrasivkörpers, also des nadelförmigen Calciumsilikats, auf, vorzugsweise 5 bis 30 Gew.-%, und noch bevorzugter 10 bis 20 Gew.-%, und noch bevorzugter ca. 15 Gew.-%.

Versuche zur Abrasivität dieser Zusammensetzungen haben ergeben, dass diese eine ausgezeichnete Reinigungswirkung, mit gleichzeitig geringer bzw. moderater Abrasivität besitzen, und damit hervorragend zur Dentalreinigung und -politur geeignet sind. Dies liegt darin begründet, dass sich das nadelförmige Calciumsilicat während der Behandlung gleichsam reduziert, wodurch ein gleichzeitiges Polieren in einem Schritt möglich wird.

Erfindungsgemäß ist der zumindest erste Abrasivkörper Wollastonit.

Wollastonit, auch Tafelspat genannt, ist ein farbloses Mineral und besitzt die chemischen Zusammensetzung CaSiO₃, bzw. genauer: Ca₃[Si₃O₉]. Chemisch gesehen handelt sich dabei um ein natürlich auftretendes Calciumsilicat bzw. das CalciumSalz der Metakieselsäure.

Gemäß einer weiteren Ausführungsform der erfindungsgemäßen Zusammensetzung enthält diese zumindest einen zweiten, nicht-nadelförmigen Abrasivkörper, wobei dieser vorzugsweise ausgewählt ist aus Feldspat, einer Kieselsäure, Bimsstein oder Hydroxylapatit, oder Kombination davon.

Feldspat gehört zu der Gruppe der Silikat-Minerale der allgemeinen chemischen Zusammensetzung (Ba,Ca,Na,K,NH₄)(Al,B,Si)₄O₈, wobei die in Klammern angegebenen Elemente sich jeweils gegenseitig vertreten können, jedoch immer im selben Mengenverhältnis zu den anderen Bestandteilen des Minerals stehen. Kieselsäuren sind Sauerstoffsäuren des Siliciums mit der allgemeinen Summenformel: [Si(OH)₂-O-]ₙ, und Hydroxylapatit ist ein Mineral aus der Klasse der Phosphate, Arsenate und Vanadate. Die vorgenannten werden erfindungsgemäß als zumindest zweiter Abrasivkörper eingesetzt und dienen der Einstellung der Grundabrasivität. Die durchschnittliche Partikelgröße D50 der eingesetzten zweiten Abrasivkörper beläuft sich dabei zwischen ca. 5 µm und 40 µm, vorzugsweise 20 bis 25 µm.

Erfindungsgemäß weist die erfindungsgemäße Zusammensetzung ferner zumindest einen Polierkörper auf, der ausgewählt ist aus Hydroxylapatit, Feldspat, Bimsstein, Eisenglimmer, wobei besonders bevorzugt ist, wenn der Polierkörper Hydroxylapatit ist.

Mit der Zugabe von Hydroxylapatit in die erfindungsgemäße Zusammensetzung wird neben dem ersten Abrasivkörper eine Substanz von ähnlicher Härte wie das native Hydroxylapatit gegeben. Dies trägt zu guten Poliereigenschaften wie auch zur Regeneration der Zahnhartsubstanz bei, wodurch wiederum bekanntermaßen eine Desensibilisierung der Zähne erreicht werden kann.

Im Gegensatz zur Verwendung als Abrasivkörper, der eine abrasive Wirkung auf die Zahnoberflächen ausübt und eine entsprechende Partikelform aufweist, wird in der erwähnten Ausführungsform erfindungsgemäß Hydroxylapatit als Polierkörper eingesetzt, welcher keinen Abrieb bewirkt.

Dabei wird dem Fachmann aus seinem Fachwissen und auf Grundlage dieser Offenbarung klar sein, in welcher Form und/oder Partikelgröße eine Substanz, bspw. Hyroxylapatit als Abrasivkörper oder als Polierkörper wirkt.

Es versteht sich, dass die erfindungsgemäße Zusammensetzung auch weitere Inhaltsstoffe aufweisen kann, wie bspw. eines oder mehrere einer Grundlage, eines Feuchthaltemittels, eines Verdickungsmittels, eines Emulgators, eines Aromas, eines Fluorids, eines Pigments und/oder eines Konservierungsmittels.

Der Einsatz dieser weiteren Inhaltsstoffe ist im Stand der Technik bereits seit längerem bekannt und erprobt, und es wird im Ermessen des Fachmanns liegen, die jeweils geeigneten Inhaltsstoffe auf Basis der hierin offenbarten Erfindung zusammenzustellen. Eine beispielhafte, nicht limitierende Aufzählung geeigneter Substanzen finden sich bspw. in der EP 2 730178 A1 und in den darin zitierten Druckschriften.

Das Aroma bzw. der Aromastoff kann insbesondere künstlich oder natürlich sein, und ist vorzugsweise ein Orangen- oder Minzaroma. Als Fluorid kann jedes im Dentalbereich geeignete Fluorid eingesetzt werden, wobei Natrium- und Kaliumfluorid bevorzugt sind. Als Feuchthaltemittel und/oder Grundlage kann bspw. Glycerin dienen, wobei auch Polydextrose und/oder Sorbitol geeignet sein können, also jedes Feuchthaltemittel, das das Austrocknen von Lebensmitteln verhindert, indem bei der Herstellung der Zusammensetzung zugesetztes Wasser gebunden wird. Beispielhafte Verdickungsmittel sind Carrageen, Xanthan, sowie Carboxymethylcellulose, wobei bspw. auch andere Cellulosen, wie Hydroxypropyl(methyl)cellulose geeignet sein können, mithin also jedes für den dentalen Bereich geeignete Verdickungsmittel, das in der Lage ist, Wasser zu binden und die Viskosität zu erhöhen. Als Emulgator kann jede Substanz/Verbindung dienen, die zwei nicht oder nur schwer miteinander mischbare Fluide, zu einem fein verteilten Gemisch (Emulsion), zu vermengen und zu stabilisieren. Beispielhaft kann als Emulgator Olefinsulfonat eingesetzt werden.

Entsprechend weist eine Ausführungsform der erfindungsgemäßen Zusammensetzung folgende konkretere Zusammensetzung auf:
5 bis 70 Gew.-%, vorzugsweise 5 bis 30 Gew.-%, noch bevorzugter 10 bis 20 Gew.-%, Wollastonit,
0 bis 50 Gew.-% eines zweiten Abrasivkörpers ausgewählt aus Feldspat, Kieselsäure, Bimsstein oder Hydroxylapatit oder einer Kombination von eines oder mehreren davon,
10 bis 50 Gew.-%, vorzugsweise 15 bis 30 Gew.-%, noch bevorzugter 18 bis 24% Gew.-% Glycerin,
10 bis 50 Gew.-%, vorzugsweise 18 bis 25 Gew.-% Wasser,
0 bis 50 Gew.-%, vorzugsweise 0 bis 25 Gew.-% eines Polierkörpers, er ausgewählt ist aus Hydroxylapatit oder Feldspat, oder Mischungen davon,
0 bis 10 Gew.-%, vorzugsweise 0 bis 1 Gew.-% eines Emulgators,
0 bis 2 Gew.-% Aroma, insbesondere Orangenaroma oder Minzaroma,
0 bis 5 Gew.-%, vorzugsweise 0 bis 1,5 Gew.-% eines Farbstoffs,
0 bis 1 Gew.-%, vorzugsweise 0,5 bis 1 Gew.-% eines Konservierungsmittels, insbesondere Phenoxyethanol oder eine Phenoxyethanol/Paraben-Mischung, und
0 bis 0,5 Gew.-% eines Fluorids, insbesondere Natriumfluorid oder Kaliumfluorid, worin die Summe aller Anteile 100 Gew.-% ergibt.

Die oben genannte Zusammensetzung kann darüber hinaus auch noch ein Verdickungsmittel, bspw. Carboxymethylcellulose, oder Kombinationen zweier oder mehrerer verschiedener Verdickungsmittel, in einer Menge von 0 bis 5,0 Gew.-% aufweisen. Ferner kann sie noch vorzugsweise 20 bis 40 Gew.-% Feldspat als zweiten Polierkörper aufweisen.

Insbesondere betrifft die vorliegende Erfindung auch die folgende bevorzugte Ausführungsform der erfindungsgemäßen Zusammensetzung:
15 Gew.-% Wollastonit als erster Abrasivkörper,
18 bis 24 Gew.-% Glycerin,
18 bis 25 Gew.-% Wasser,
0 bis 25 Gew.-% Hydroxylapatit als ersten Polierkörper,
20 bis 40 Gew.-% Feldspat als zweiten Polierkörper
0 bis 1 Gew.-% eines Emulgators,
0 bis 2 Gew.-% Aroma, insbesondere Orangenaroma oder Minzaroma,
0 bis 1,5 Gew.-% eines Farbstoffs oder Farbstoffgemischs,
0 bis 1 Gew.-% Phenoxyethanol oder eine Phenoxyethanol/Paraben-Mischung als Konservierungsmittel, und
0 bis 0,5 Gew.-% Fluorid, insbesondere Natriumfluorid oder Kaliumfluorid,
worin die Summe aller Anteile 100 Gew.-% ergibt.

Schließlich betrifft die vorliegende Erfindung auch die Verwendung einer wie oben erläuterten erfindungsgemäßen Zusammensetzung zur Dentalpolitur und/oder - reinigung, insbesondere in der professionalen Zahnreinigung/-politur in Zahnarztpraxen oder Zahnklinken. Dabei versteht sich, dass die Anwendungen der erfindungsgemäßen Zusammensetzung auch im privaten Bereich, ggf. nach entsprechender Einweisung durch zahnmedizinisch geschultes Personal, vorgenommen werden kann.

Vorliegend wird dabei unter "Dentalpolitur" jede Anwendung bzw. Verwendung der erfindungsgemäßen Zusammensetzung verstanden, im Rahmen welcher dentale Oberflächen poliert oder gereinigt werden. Entsprechend umfasst der Begriff "Zahnoberflächen" vorliegend nicht nur die Oberflächen natürlicher Zähne, sondern auch die von Zahnersatz bzw. Implantaten.

In einem erfindungsgemäßen Verfahren wird die erfindungsgemäße Zusammensetzung gemäß der vorliegenden Erfindung auf die Zahnoberflächen mittels eines geeigneten Applikators, bspw. einem Bürstchen oder Gummikelches, aufgetragen, ggf. nachdem zuvor mittels geeigneter Gerätschaften, wie bspw. Pulverstrahlgeräten und/oder Handgeräten, hartnäckiger Zahnbelag entfernt wurde. Mittels der dann aufgetragenen Polierpaste können zusätzlich Verfärbungen entfernt werden, sowie die Zahnoberfläche "geglättet", bzw. Risse und Löcher aufgefüllt werden, wodurch die Zahnoberfläche glatt, also "poliert", erscheint.

Durch den ggf. ebenfalls vorgesehenen Zusatz eines Fluorids kann die Remineralisierung der Zahnoberflächen beschleunigt werden.

Es versteht sich, dass die vorstehend genannten und nachstehend noch zu erläuternden Merkmale nicht nur in der jeweils angegebenen Kombination, sondern auch in Alleinstellung verwendbar sind, ohne den Rahmen der vorliegenden Erfindung zu verlassen.

Weitere Vorteile ergeben sich aus der nachstehenden Beschreibung und den hierzu durchgeführten Versuchen und Ausführungsbeispielen sowie in den Figuren. Es zeigt:

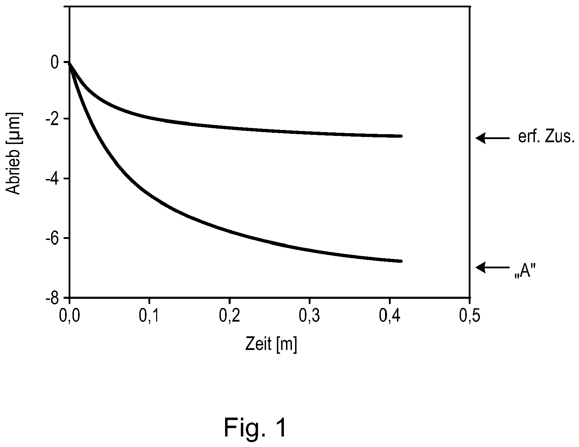

- Fig. 1: die diagrammatische Darstellung des zeitlichen Verlaufs des Abriebs bei Anwendung einer erfindungsgemäßen Zusammensetzung (obere Kurve), sowie einer Vergleichspaste "A" (untere Kurve);

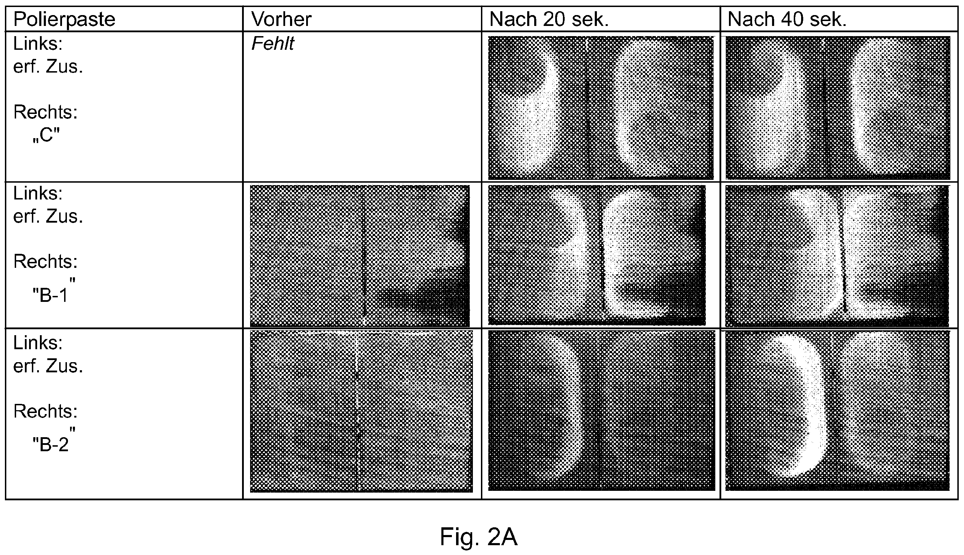

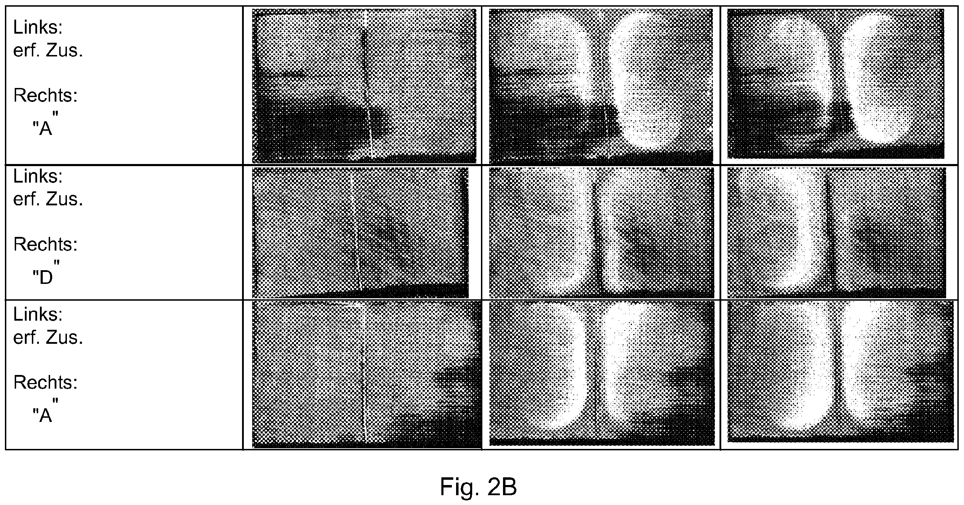

- Fig. 2: Vergleichsaufnahmen von zuvor in schwarzen Tee eingelegten Elfenbeinplättchen, die jeweils einmal mit einer Ausführungsform der erfindungsgemäßen Zusammensetzung behandelt (poliert/gereinigt) wurden (jeweils rechter Teil im Plättchen), oder mit im Handel erhältlichen Vergleichspasten (jeweils linker Teil der Elfenbeinplättchen); **A:** Vergleichsprodukte "B-1", "B-2" und "C"; **B:** Vergleichsprodukte "A" und "D".

### Beispiele

Beispielhafte Ausführungsformen von erfindungsgemäßen Zusammensetzungen wurden wie folgt erstellt:

### Zusammensetzung Polierpaste

| **Bestandteil** | **Anteil [%]** | **Zweck** |
|---|---|---|
| Wollastonit | 15 | Abrasivkörper (Nadelförmig) |
| Glycerin | 23 - 28 | Feuchthaltemittel / Grundlage |
| Wasser | 15-20 | Grundlage |
| Hydroxylapatit (HAP) | 5-30 | Polierkörper |
| Feldspat | 20 - 40 | Polierkörper |
| Emulgator | 0 - 1 | Konsistenz und haptisches Verhalten |
| ggf. Orangen- oder Minzaroma | 0-2 | Aroma + Süßung |
| Farbpigment | 0-2 | Farbe mit Talkum |
| Phenoxyethanol | 0,5 | Konservierung |
| Natriumfluorid | 0-0,5 | Fluoridierung |

Die o.g. Zusammensetzungen wurde gemäß üblichen Formulierungsverfahren hergestellt, wobei auch Zusammensetzungen ohne Minzaroma und hergestellt wurden.

Ferner wurde eine Pasten-Zusammensetzung getestet, die folgende Inhaltsstoffe umfasste: Wollastonit 15 Gew.-%, Glycerin, 20 Gew.-%, Konservierungsmittel (Mischung aus Phenoxyethanol und Paraben) 0,8 Gew.-%, Verdickungsmittel 0,6 Gew.-%, Emulgator 0,15 Gew.-%, Natriumfluorid, 0,15 Gew.-%, Hydroxylapatit 5 Gew.-%, Feldspat 35 Gew.-%, Aroma 0,09 Gew.-%, 1,4 Gew.-% Farbe, wobei die fehlenden Gew.-% mit Wasser als Grundlage Verdünner aufgefüllt wurden.

### Untersuchungen zum Abrieb

Mit der beispielhaften erfindungsgemäßen Paste, sowie mit zwei Vergleichspasten (Produkt "A": basierend insbesondere auf Perlit, Natriumfluorid, Titandioxid, Glycerin, Wasser, Aroma, Sorbitol Silica; sowie Produkte "B 1 bis 3": basierend insbesondere auf Sorbitol, Xylitol, Aminfluorid, Siliziumdioxid, Bimsstein, Titandioxid, Wasser, Farbstoff und Aroma; in den Abrasionsstärken mittel, hoch und niedrig) wurde ein im Handel (bspw. über Vita, Bad Säckingen, Deutschland) erhältlicher Keramikblock behandelt. Als Vergleich diente darüber hinaus ein unbehandelter Keramikblock.

Die Blöcke wurden jeweils mit den Polierpasten unter Verwendung eines handelsüblichen Hand- und Winkelstücks behandelt. Die Politur erfolgt mit einem ebenfalls handelsüblichen Polierkelch unter den folgenden Bedingungen: Anpressdruck 200g, Auslenkung des Probentisches. 10°, Polierdauer: 15 s bei 2000 rpm.

Die Oberflächen wurden nachfolgend rasterelektronenmikroskopisch (REM) untersucht (REM-Abbildungen nicht gezeigt).

Ähnliche Ergebnisse konnten bei der Behandlung von Implantaten erzielt werden (REM-Abbildungen nicht gezeigt).

Die Untersuchungen mittels Rasterelektronenmikroskop ergaben, dass für die mit einer Ausführungsform der erfindungsgemäßen Polierpasten-Zusammensetzung behandelten Oberflächen im Vergleich zu einer unbehandelten Oberfläche kein Abrieb beobachtet werden konnte.

Fig. 1 zeigt darüber hinaus eine diagrammatische Darstellung der mittels eines Tribometers (auf Polymethylmethacrylat, PMMA) gemessenen Abriebsunterschiede der erfindungsgemäßen Zusammensetzung im Vergleich zu herkömmlichen Polierpasten: Die obere Kurve in Fig. 1 zeigt den Verlauf des Abriebs über die Zeit bei Anwendung der erfindungsgemäßen Zusammensetzung, die untere Kurve - und damit einen größeren Abrieb zeigt den Verlauf des Abriebs über die Zeit bei Anwendung des Vergleichsproduktes "A". Dies zeigte, dass die erfindungsgemäßen Polierpasten deutlich zahnschonender sind als die beiden im Handel erhältlichen Vergleichspasten.

### Untersuchungen zur Reinigungswirkung

Im Weiteren wurden auch Untersuchungen zur Reinigungswirkung durchgeführt; hierzu wurde jeweils die Reinigungswirkung der erfindungsgemäßen Zusammensetzung ("erf. Zus.") sowie zweier im Handel erhältlicher Vergleichsprodukte an Elfenbeinplättchen (12 x 20 mm) untersucht, die zuvor 24 Stunden in schwarzen Tee eingelegt wurden. Die so vorbehandelten Elfenbeinplättchen wurden in einer Laborvorrichtung fixiert.

Jeweils 0,1 g Polierpaste wurde in einen Polierpastenkelch (Kerr Hawe soft; Kerrdental, Rastatt, Deutschland) gegeben und dieser mit konstantem Anpressdruck (200 g) jeweils 20 Sekunden auf die Elfenbeinplättchen gepresst, wobei der Einspanntisch mit einer konstanten Winkelgeschwindigkeit bewegt wurde.

Als Vergleichspasten wurden hier die bereits oben genannte Produkte "A" und "B" (mit mittlerer Abrasivität "B-1" und hoher Abrasivität "B-2"), sowie zwei weitere Vergleichsprodukte "C": basierend insbesondere auf Bimsstein, Glycerin, Cellulose, Titandioxid, Natriumfluorid, Wasser; und "D": basierend auf den abrasiven Mineralen Bimsstein, Aluminiumoxid, Calciumcarbonat; Feuchthaltemittel, Bindemittel, Aromastoffe, Methylparaben, getestet.

Die Reinigungswirkung, die optisch bestimmt wurde, erwies sich bei der erfindungsgemäßen Zusammensetzung/Paste teilweise besser als bei den Vergleichspasten, zumindest aber gleichwertig, wobei die Ergebnisse hierzu in Fig. 2A und 2B gezeigt sind: In den Abbildungen sind die Plättchen jeweils vor der Behandlung, nach 20 sek. und nach 40 sek. Behandlung gezeigt, wobei jeweils im linken Teil des Elfenbeinplättchens mit einer Ausführungsform der erfindungsgemäßen Zusammensetzung gereinigt wurde, und jeweils im rechten Teil des Elfenbeinplättchens mit einem im Handel erhältlichen Vergleichsprodukt.

Die dargestellten Ergebnisse zeigen daher, dass die erfindungsgemäße Zusammensetzung deutlich zahnschonender ist, jedoch gleichzeitig eine bessere Reinigungswirkung besitzt als die bisher im Handel erhältlichen Zahnreinigungs- bzw. Polierpasten.

## Patentansprüche

1. Zusammensetzung zur Dentalpolitur und/oder -reinigung, umfassend 5 bis 70 Gew.-% eines nadelförmigen kristallinen Calciumsilikats als ein erster Abrasivkörper, worin das nadelförmige kristalline Calciumsilikat ein Längen-Breiten-Verhältnis von 3 : 1 bis 20 : 1 aufweist, und wobei das nadelförmige kristalline Calciumsilikat Wollastonit ist, und wobei ein Polierkörper vorliegt, der ausgewählt ist aus Hydroxylapatit, Feldspat, Bimsstein, Eisenglimmer.

2. Zusammensetzung zur Dentalpolitur und/oder -reinigung nach Anspruch 1, worin zumindest ein zweiter, nicht-nadelförmiger Abrasivkörper vorliegt.

3. Zusammensetzung zur Dentalpolitur und/oder -reinigung nach Anspruch 2, **dadurch gekennzeichnet, dass** der zumindest zweite nicht-nadelförmige Abrasivkörper ausgewählt ist aus Feldspat, Kieselsäure, Bimsstein oder Hydroxylapatit, oder Kombinationen davon.

4. Zusammensetzung zur Dentalpolitur und/oder -reinigung nach einem der vorstehenden Ansprüche, worin ferner eines oder mehrere einer Grundlage, eines Verdickungsmittels, Emulgators, Aromas, Pigments und/oder Konservierungsmittels vorliegen.

5. Zusammensetzung zur Dentalpolitur und/oder -reinigung nach einem der vorstehenden Ansprüche, umfassend
5 bis 70 Gew.-% Wollastonit als erster Abrasivkörper,
0 bis 50 Gew.-% eines zweiten Abrasivkörpers ausgewählt aus Feldspat, Kieselsäure, Bimsstein oder Hydroxylapatit oder einer Kombination davon,
10 bis 50 Gew.-% Glycerin,
10 bis 50 Gew.-% Wasser,
0 bis 50 Gew.-% eines Polierkörpers, der ausgewählt ist aus Hydroxylapatit und Feldspat, oder Mischungen davon,
0 bis 10 Gew.-% eines Emulgators,
0 bis 2 Gew.-% Aroma,
0 bis 5 Gew.-% eines Farbstoffs,
0 bis 1 Gew.-% eines Konservierungsmittels, oder eines Gemisches von Konservierungsmitteln und
0 bis 0,5 Gew.-% Natriumfluorid oder Kaliumfluorid,
worin die Summe aller Anteile 100 Gew.-% ergibt.

6. Zusammensetzung nach einem der vorstehenden Ansprüche, **dadurch gekennzeichnet, dass** sie Folgendes aufweist:
10 bis 20 Gew.-% Wollastonit als erster Abrasivkörper,
18 bis 24 Gew.-% Glycerin,
18 bis 25 Gew.-% Wasser,
0 bis 25 Gew.-% Hydroxylapatit als ersten Polierkörper,
20 bis 40 Gew.-% Feldspat als zweiten Polierkörper
0 bis 1 Gew.-% eines Emulgators,
0 bis 2 Gew.-% Orangenaroma oder Minzaroma,
0 bis 1,5 Gew.-% eines Farbstoffs,
0 bis 1 Gew.-% Phenoxyethanol oder eine Phenoxyethanol/Paraben-Mischung als Konservierungsmittel, und
0 bis 0,5 Gew.-% Natriumfluorid oder Kaliumfluorid,
worin die Summe aller Anteile 100 Gew.-% ergibt.

7. Zusammensetzung nach einem der Ansprüche 1 bis 6, **dadurch gekennzeichnet, dass** sie ca. 15 Gew.-% Wollastonit als erster Abrasivkörper aufweist.

8. Zusammensetzung nach einem der vorstehenden Ansprüche zur Verwendung bei der Politur und/oder Reinigung von natürlichen oder künstlichen Zahnoberflächen.

## Claims

1. A composition for dental polishing and/or cleaning, comprising 5 to 70 % by weight of an acicular crystalline calcium silicate as a first abrasive body, the acicular crystalline calcium silicate having a length-to-width ratio of from 3:1 to 20:1, and wherein the acicular crystalline calcium silicate is wollastonite, and wherein a polishing body is present, which is selected from hydroxylapatite, feldspar, pumice, micaceous iron oxide.

2. The composition for dental polishing and/or cleaning as claimed in claim 1, wherein at least a second, nonacicular abrasive body is present.

3. The composition for dental polishing and/or cleaning as claimed in claim 2, **characterized in that** the at least second nonacicular abrasive body is selected from feldspar, silicic acid, pumice or hydroxylapatite, or combinations thereof.

4. The composition for dental polishing and/or cleaning as claimed in any of the preceding claims, wherein one or more of a base, of a thickener, of an emulsifier, of a flavor, of a pigment and/or of a preservative are further present.

5. The composition for dental polishing and/or cleaning as claimed in any of the preceding claims, comprising
from 5 to 70% by weight of wollastonite as first abrasive body,
from 0 to 50% by weight of a second abrasive body selected from feldspar, silicic acid, pumice or hydroxylapatite or a combination thereof,
from 10 to 50% by weight of glycerol,
from 10 to 50% by weight of water,
from 0 to 50% by weight of a polishing body selected from hydroxylapatite and feldspar, or mixtures thereof,
from 0 to 10% by weight of an emulsifier,
from 0 to 2% by weight of flavor,
from 0 to 5% by weight of a dye,
from 0 to 1% by weight of a preservative, or of a mixture of preservatives, and
from 0 to 0.5% by weight of sodium fluoride or potassium fluoride,
the sum of all fractions yielding 100% by weight.

6. The composition as claimed in any of the preceding claims, **characterized in that** it comprises the following:
from 10 to 20% by weight of wollastonite as first abrasive body,
from 18 to 24% by weight of glycerol,
from 18 to 25% by weight of water,
from 0 to 25% by weight of hydroxylapatite as first polishing body,
from 20 to 40% by weight of feldspar as second polishing body,
from 0 to 1% by weight of an emulsifier,
from 0 to 2% by weight of orange flavor or mint flavor,
from 0 to 1.5% by weight of a dye,
from 0 to 1% by weight of phenoxyethanol or a phenoxyethanol/paraben mixture as preservative, and
from 0 to 0.5% by weight of sodium fluoride or potassium fluoride,
the sum of all fractions yielding 100% by weight.

7. The composition as claimed in any of claims 1 to 6, **characterized in that** it comprises approximately 15% by weight of wollastonite as first abrasive body.

8. The composition as claimed in any of the preceding claims for use in polishing and/or cleaning natural or artificial tooth surfaces.

## Revendications

1. Composition pour le polissage et/ou le nettoyage dentaire, comprenant 5 à 70 % en poids d'un silicate de calcium cristallin aciculaire en tant que premier corps abrasif, le silicate de calcium cristallin aciculaire présentant un rapport longueur-largeur de 3:1 à 20:1, et le silicate de calcium cristallin aciculaire étant de la wollastonite, et un corps de polissage étant présent, qui est choisi parmi l'hydroxylapatite, le feldspath, la pierre ponce, le mica de fer.

2. Composition pour le polissage et/ou le nettoyage dentaire selon la revendication 1, dans laquelle au moins un deuxième corps abrasif non aciculaire est présent.

3. Composition pour le polissage et/ou le nettoyage dentaire selon la revendication 2, **caractérisée en ce que** ledit au moins un deuxième corps abrasif non aciculaire est choisi parmi le feldspath, la silice, la pierre ponce ou l'hydroxylapatite, ou les combinaisons de ceux-ci.

4. Composition pour le polissage et/ou le nettoyage dentaire selon l'une quelconque des revendications précédentes, dans laquelle un ou plusieurs composants choisis parmi une base, un épaississant, un émulsifiant, un arôme, un pigment et/ou un conservateur sont en outre présents.

5. Composition pour le polissage et/ou le nettoyage dentaire selon l'une quelconque des revendications précédentes, comprenant :
5 à 70 % en poids de wollastonite en tant que premier corps abrasif,
0 à 50 % en poids d'un deuxième corps abrasif choisi parmi le feldspath, la silice, la pierre ponce ou l'hydroxylapatite, ou une combinaison de ceux-ci,
10 à 50 % en poids de glycérine,
10 à 50 % en poids d'eau,
0 à 50 % en poids d'un corps de polissage, qui est choisi parmi l'hydroxylapatite et le feldspath, ou les mélanges de ceux-ci,
0 à 10 % en poids d'un émulsifiant,
0 à 2 % en poids d'un arôme,
0 à 5 % en poids d'un colorant,
0 à 1 % en poids d'un conservateur, ou d'un mélange de conservateurs, et
0 à 0,5 % en poids de fluorure de sodium ou de fluorure de potassium,
la somme de toutes les proportions étant de 100 % en poids.

6. Composition selon l'une quelconque des revendications précédentes, **caractérisée en ce qu'**elle comprend les composants suivants :
10 à 20 % en poids de wollastonite en tant que premier corps abrasif,
18 à 24 % en poids de glycérine,
18 à 25 % en poids d'eau,
0 à 25 % en poids d'hydroxylapatite en tant que premier corps de polissage,
20 à 40 % en poids de feldspath en tant que deuxième corps de polissage,
0 à 1 % en poids d'un émulsifiant,
0 à 2 % en poids d'un arôme d'orange ou d'un arôme de menthe,
0 à 1,5 % en poids d'un colorant,
0 à 1 % en poids de phénoxyéthanol ou d'un mélange de phénoxyéthanol/parabène en tant que conservateur, et
0 à 0,5 % en poids de fluorure de sodium ou de fluorure de potassium,
la somme de toutes les proportions étant de 100 % en poids.

7. Composition selon l'une quelconque des revendications 1 à 6, **caractérisée en ce qu'**elle comprend environ 15 % en poids de wollastonite en tant que premier corps abrasif.

8. Composition selon l'une quelconque des revendications précédentes, destinée à une utilisation lors du polissage et/ou du nettoyage de surfaces de dents naturelles ou artificielles.
